# EUROPEAN PATENT SPECIFICATION

(11) **EP 3 345 972 B1**
(45) Date of publication and mention of the grant of the patent: **19.06.2024**
(21) Application number: 16841365.6
(22) Date of filing: 29.07.2016
(51) Int. Cl.: C08L 101/12, B82Y 5/00, C08K 3/04, C08L 71/08, C08L 81/04

(54) **RESIN COMPOSITION**
HARZZUSAMMENSETZUNG
COMPOSITION DE RÉSINE

(30) Priority: 01.09.2015 JP 2015171970; 19.01.2016 JP 2016007949; 29.03.2016 JP 2016065891
(43) Date of publication of application: 11.07.2018
(73) Proprietor: Daicel Corporation, Osaka-shi, Osaka 530-0011 (JP)
(72) Inventor: ITO, Hisayoshi, Himeji-shi Hyogo 671-1283 (JP); KUME, Atsushi, Himeji-shi Hyogo 671-1283 (JP)
(74) Representative: Grünecker Patent- und Rechtsanwälte PartG mbB
(86) International application number: PCT/JP2016/072247
(87) International publication number: WO 2017/038333

(56) References cited:
- WO-A1-2014/049212
- WO-A2-2015/097347
- CN-A- 102 757 635
- JP-A- 2004 051 937
- JP-A- 2012 161 965
- JP-A- 2012 170 913
- JP-A- 2015 127 364
- US-A1- 2014 091 253
- DATABASE WPI Week 201357 Thomson Scientific, London, GB; AN 2013-B37921 XP002791302, -& CN 102 757 635 A (SHANGHAI GENIUS ADVANCED MATERIAL CO LTD) 31 October 2012 (2012-10-31)
- DATABASE WPI Week 201259 Thomson Scientific, London, GB; AN 2012-L29333 XP002791303, -& JP 2012 161965 A (VISION KAIHATSU KK) 30 August 2012 (2012-08-30)
- DENG SHULING ET AL: "Nanodiamond as an efficient nucleating agent for polyphenylene sulfide", THERMOCHIMICA ACTA, ELSEVIER SCIENCE PUBLISHERS, AMSTERDAM, NL, vol. 584, 20 May 2014 (2014-05-20), pages 51-57, XP028650723, ISSN: 0040-6031, DOI: 10.1016/J.TCA.2014.03.017
- HISAYOSHI ITO: 'Nanodiamond no Tokusei to Oyo' CONVERTECH vol. 43, no. 5, May 2015, pages 84 - 86, XP009508782
- SHULING DENG ET AL.: 'Nanodiamond as an efficient nucleating agent for polyphenylene sulfide' THERMOCHIMICA ACTA vol. 584, 20 May 2014, pages 51 - 57, XP028650723
- HISAYOSHI ITO: 'Bakugoho Nanodiamond no Kaihatsu' KAGAKU KEIZAI vol. 62, no. 12, October 2015, pages 67 - 70, XP009508866
- MASAHIRO NISHIKAWA ET AL.: 'Development of Detonation Nanodiamonds as Functional Materials' MOLTEN SALTS vol. 59, no. 2, 2016, pages 55 - 62, XP009509278

## Description

### Technical Field

The present invention relates to a resin composition which is usable typically as engineering plastics and super engineering plastics. This application relates to Japanese Patent Application No. 2015-171970, filed on September 1, 2015; Japanese Patent Application No. 2016-007949, filed on January 19, 2016; and Japanese Patent Application No. 2016-065891, filed on March 29, 2016.

### Background Art

In production of resin shaped articles, steps such as the step of softening, melting, and kneading a material resin composition and the step of shape processing the resin composition are attended with heating of the material resin composition. In such steps attended with heating, the heating may cause radicals to be generated in the resin composition, and the radicals may induce a decomposition reaction and/or a crosslinking reaction in the resin composition. These decomposition reaction and crosslinking reaction may generally result in deterioration in chemical structure and/or properties of the resin composition. In addition, the crosslinking reaction may cause the resin composition to have a higher viscosity and to offer lower workability. In order to eliminate or minimize the decomposition reaction and crosslinking reaction as above, some of resin compositions to be subjected to a step attended with heating further contain hindered phenolic compounds as radical stabilizers, where the hindered phenolic compounds functionally trap (scavenge) and/or stabilize radicals. Such hindered phenolic compounds are described typically in Patent Literature (PTL) 1, PTL 2, and PTL 3 below.

PTL 4 relates to a nanodiamond containing thermoplastic thermal composite which comprises 0.01 to 80 wt% of nanodiamond particles, 1 to 90 wt% of filler, and 5 to 80 wt% of thermoplastic polymer.

PTL 5 describes a composite material comprising 64.5 to 89.3 parts by weight of polyether ether ketone, and 0.5 to 5 parts by weight of nanodiamond powder.

PTL 6 relates to a diamond-resin compound material in which fine diamond particles with a median diameter of 2 to 250 nm are dispersed in a resin.

PTL 7 describes a composite material comprising nanodiamond and at least one filler and at least one polymer.

Deng Shuling et al. relates to a composite material containing nanodiamond particles compounded in PPS.

### Citation List

### Patent Literature

PTL 1: Japanese Unexamined Patent Application Publication (JP-A) No. 2002-332306
PTL 2: JP-A No. 2006-160809
PTL 3: JP-A No. 2011-208157
PTL 4: US 2014/0091253 A1
PTL 5: CN 102 757 635 A
PTL 6: JP 2012-161965 A
PTL 7: WO 2015/097347 A2

### Non-Patent Literature

Deng Shuling et al., "Nanodiamond as an efficient nucleating agent for polyphenylene sulfide", Thermochemica Acta, vol. 584, pages 51-57

### Summary of Invention

### Technical Problem

Engineering plastics, super engineering plastics, and other highly heat-resistant resin materials have high melting points and require correspondingly high working temperatures in their shape processing. In particular, some shape processing of super engineering plastics requires high working temperatures even of, for example, 280°C or higher. However, most of radical stabilizers, which are organic compounds such as hindered phenolic compounds, cannot endure high temperatures as to be required in shape processing of engineering plastics and other highly heat-resistant resin materials, and are decomposed at high temperatures typically of 280°C or higher. This causes technical difficulty in shape processing of highly heat-resistant resin materials or resin compositions, which require high working temperatures, while restraining deterioration and thickening in working with heating.

The present invention has been made under these circumstances and has an object to provide a resin composition which is suitable for offering high heat resistance while restraining deterioration and thickening in working with heating.

### Solution to Problem

The subject matter of the invention is as set out in the appended claims.

The present invention provides, according to one embodiment, a resin composition. This resin composition contains a thermoplastic resin and nanodiamond particles. The thermoplastic resin has a melting point of 280°C or higher or a glass transition temperature of 220°C or higher. The nanodiamond particles comprise surface hydroxy groups, wherein the nanodiamond particles have a proportion of hydroxy-bonded carbon of 16.8% or more of the totality of carbon in the nanodiamond particles. In the present invention, the nanodiamond particles may be any of nanodiamond primary particles and nanodiamond secondary particles, where the nanodiamond secondary particles result from aggregation of primary particles. As used herein, the term "nanodiamond primary particle" refers to a nanodiamond having a particle diameter of 10 nm or less. Also as used herein, the term "glass transition temperature" refers to a value measured by differential scanning calorimetry (DSC) in conformity to the Japanese Industrial Standard (JIS) (JIS K 7121: Testing Methods for Transition Temperatures of Plastics).

The resin composition according to the embodiment contains a thermoplastic resin having a melting point of 280°C or higher or a glass transition temperature of 220°C or higher. To soften/melt the thermoplastic resin so as to knead or shape the resin composition, heating should be performed up to a high temperature significantly higher than the glass transition temperature of the thermoplastic resin. Even if such heating causes radicals to be generated in the resin composition, the decomposition reaction and crosslinking reaction in the thermoplastic resin are restrained by the presence of the nanodiamond particles in the resin composition, where the reactions will be caused by the action of the radicals. This is probably because as follows. Nanodiamonds can offer heat resistance even in an environment at a high temperature of higher than 450°C. A graphite layer is assumed to be formed on at least part of, namely on at least the {111} face of, the nanodiamond primary particles each of which is a nanodiamond particle itself or is contained in a nanodiamond particle, where the graphite layer results from spontaneous transition from sp3 carbon (carbon with sp3 structure) which formerly constitutes the diamonds themselves. The presence of sp² carbon (carbon with sp² structure) in the graphite layer probably contributes in part to trapping/stabilization of radicals, and this probably restrains decomposition and crosslinking, namely, deterioration of the thermoplastic resin, where the decomposition and crosslinking will be caused by the action of the radicals. In addition, the restrainment of the crosslinking reaction tends to restrain the thickening of the thermoplastic resin, namely, the thickening of the resin composition during working with heating. The restrainment of the decomposition reaction and the crosslinking reaction in the resin composition during working with heating contributes to restraining deterioration of the resin composition in heat resistance and to offering high heat resistance of the resin composition, where the resin composition contains the thermoplastic resin having a melting point of 280°C or higher or a glass transition temperature of 220°C or higher. As described above, the resin composition is suitable for offering high heat resistance while restraining deterioration and thickening during working with heating.

Preferably, the resin composition contains the nanodiamond particles in an amount of 0.001 to 5 parts by weight per 100 parts by weight of the thermoplastic resin. With an increasing amount of the nanodiamond particles, the resin composition can enjoy significant advantageous effects of the nanodiamond particles, which serve as a radical stabilizer. The resin composition may preferably contain the nanodiamond particles in an amount of 5 parts by weight or less, from the viewpoint of allowing the thermoplastic resin in the resin composition to appropriately offer its functions.

Preferably, the nanodiamond particles are detonation nanodiamond particles (nanodiamond particles formed by a detonation technique). The detonation technique enables appropriate formation of nanodiamonds having a primary particle diameter of 10 nm or less.

The nanodiamond particles include surface hydroxy groups. Preferably, the nanodiamond particles include surface carboxy groups. Preferably, the nanodiamond particles include surface carbonyl groups. These surface functional groups are considered to co-operate with sp² carbon present on the nanodiamond surfaces to form a conjugated system, to contribute to trapping and stabilization of radicals, and to thereby contribute to restrainment of the decomposition and crosslinking, namely, deterioration of the thermoplastic resin, where the decomposition and crosslinking will be caused by the action of the radicals.

The nanodiamond particles have a particle diameter D50 (median diameter) of preferably 10 um or less, more preferably 500 nm or less, and furthermore preferably 200 nm or less. The nanodiamond particles for use in the present invention are any of nanodiamond primary particles and secondary particles, where the secondary particles result from the aggregation of such primary particles. The nanodiamond particles preferably have a particle diameter D50 of 10 µm or less, from the viewpoints of allowing the nanodiamond particles to surely have a sufficient surface area per unit mass and to thereby efficiently offer functions as a radical stabilizer.

Preferably, the thermoplastic resin includes at least one of an aromatic poly(ether ketone) and a poly(phenylene sulfide). The aromatic poly(ether ketone) may typically include at least one selected from the class consisting of poly(ether ketone)s, poly(ether ether ketone)s, poly(ether ketone ketone)s, and poly(ether ether ketone ketone)s. The configuration as above is suitable or advantageous for allowing the resin composition to actually have, in addition to high heat resistance, other properties such as high flame retardancy, high wear resistance, and high chemical resistance.

### Brief Description of Drawings

FIG. 1 is an enlarged schematic view of a resin composition according to one embodiment of the present invention; and
FIG. 2 is a process chart of a method according to an embodiment for producing nanodiamonds which are usable as the nanodiamond particles contained in the resin composition illustrated in FIG. 1.

### Description of Embodiments

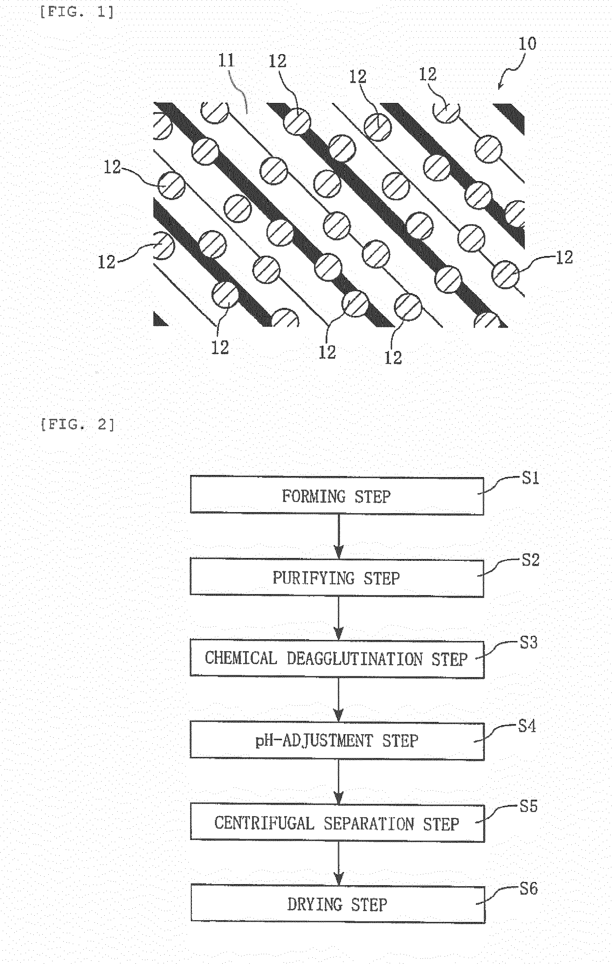

FIG. 1 is an enlarged schematic view of the resin composition 10 according to one embodiment of the present invention. The resin composition 10 contains a thermoplastic resin 11 and nanodiamond particles (ND particles) 12. The resin composition 10 can be in any form, such as a pellet or another resin shaping material form, a softened or melted article form derived from the resin shaping material form, and a resin shaped article form shaped through the softened/melted state.

The thermoplastic resin 11 is a principal material in the resin composition 10 to offer functions such as heat resistance and has a melting point of 280°C or higher or a glass transition temperature of 220°C or higher. Such thermoplastic resins generally include crystalline thermoplastic resins and amorphous thermoplastic resins. The category "thermoplastic resin" 11 for use in the embodiment includes crystalline thermoplastic resins having a melting point of 280°C or higher or a glass transition temperature of 220°C or higher; and amorphous thermoplastic resins having a glass transition temperature of 220°C or higher. Non-limiting examples of the thermoplastic resin 11 as above include aromatic poly(ether ketone)s, poly(phenylene sulfide)s (melting point: 280°C, glass transition temperature: 90°C), poly(ether sulfone)s (glass transition temperature: 225°C), polyarylates (glass transition temperature: 275°C), polyamideimides (glass transition temperature: 275°C), thermoplastic polyimides (glass transition temperature: 250°C), polybenzimidazoles (glass transition temperature: 427°C), polyamide 9T (melting point: 306°C, glass transition temperature: 125°C), and liquid-crystal polymers having a melting point of 280°C or higher. These are also called super engineering plastics or engineering plastics. The thermoplastic resin 11 preferably contains at least one of an aromatic poly(ether ketone) and a poly(phenylene sulfide). This is preferred for allowing the resin composition 10, which contains the thermoplastic resin 11, to actually have high heat resistance, high flame retardancy, high wear resistance, and high chemical resistance. The aromatic poly(ether ketone) may be selected typically from the class consisting of poly(ether ketone)s (melting point: 373°C, glass transition temperature: 140°C), poly(ether ether ketone)s (melting point: 334°C, glass transition temperature: 143°C), poly(ether ketone ketone)s (melting point: 396°C, glass transition temperature: 165°C), and poly(ether ether ketone ketone)s (melting point: 360°C, glass transition temperature: 149°C). The resin composition 10 may contain each of different thermoplastic resins 11 alone or in combination.

The resin composition 10 contains the thermoplastic resin 11 in a content of typically 60 to 99.999 mass percent, and preferably 90 to 95 mass percent.

The ND particles 12 contained in the resin composition 10 is a component to offer functions as a radical stabilizer in the resin composition 10 and are any of nanodiamond primary particles, and nanodiamond secondary particles, which result from aggregation of primary particles. As used herein, the term "nanodiamond primary particle" refers to a nanodiamond having a particle diameter of 10 nm or less. Such nanodiamonds having a primary particle diameter of 10 nm or less can be formed by the so-called detonation technique. The nanodiamond particles can offer heat resistance even in an environment at a high temperature of higher than 450°C. The ND particles 12 in the resin composition 10 have a particle diameter D50 (median diameter) of preferably 10 um or less, more preferably 500 nm or less, and furthermore preferably 200 nm or less. The ND particles 12 preferably have a particle diameter D50 of 10 µm or less, from the viewpoints of allowing the ND particles 12 to surely have a sufficient surface area per unit mass and to efficiently offer functions as a radical stabilizer. The particle diameter D50 of the ND particles 12 can be measured typically by small-angle light scattering technique and by dynamic light scattering technique. For example, the particle diameter D50 of relatively large particles having a particle diameter of 1 um or more can be measured by small-angle light scattering technique. Also for example, the particle diameter D50 of relatively small particles having a particle diameter of less than 1 µm can be measured by dynamic light scattering technique.

The resin composition 10 contains the ND particles 12 in an amount of typically 0.001 to 5 parts by weight per 100 parts by weight of the totality of the thermoplastic resin 11 in the resin composition 10. With an increasing amount of the ND particles 12, the resin composition 10 can enjoy significant advantageous effects of the ND particles 12, which serve as a radical stabilizer. Thus, the lower limit of the amount of the ND particles 12 is preferably 0.002 part by weight, more preferably 0.01 part by weight, and furthermore preferably 0.1 part by weight. The resin composition 10 may preferably contain the ND particles 12 in an amount of 5 parts by weight or less, from the viewpoint of allowing the thermoplastic resin 11 in the resin composition 10 to offer functions, such as high heat resistance, appropriately. The upper limit of the amount of the ND particles 12 is preferably 3.0 parts by weight, more preferably 2.0 parts by weight, and furthermore preferably 1.0 part by weight, from the viewpoint of allowing the resin composition 10 to enjoy the advantageous effects due to the presence of a smaller amount of the ND particles 12 in the resin composition 10.

In the embodiment, the ND particles 12 have, as a surface functional group, hydroxy groups and optionally at least one type of functional group selected from the class consisting of carboxy groups, and carbonyl groups. These surface functional groups are considered to contribute to restrainment of decomposition and crosslinking, namely, deterioration of the thermoplastic resin 11, where the deterioration will be caused by the action of radicals.

The ND particles 12 in the resin composition 10 have a proportion of hydroxy-bonded carbon of 16.8% or more, preferably 17.0% or more, more preferably 18.0% or more, furthermore preferably 20.0% or more, and still more preferably 25.0% or more, of the totality of carbon in the ND particles 12. The upper limit of the proportion of the hydroxy-bonded carbon is typically 40.0%. The nanodiamonds have a basic skeleton constituted by sp³ carbon, as with bulk diamond. As used herein, the term "hydroxy-bonded carbon" refers to carbon to which a hydroxy group (-OH) is bonded, where the hydroxy group is attached on the basis skeleton of a nanodiamond and present at the surface of the nanodiamond. In the embodiment, the proportion of the hydroxy-bonded carbon is defined as a value determined by solid-state ¹³C-NMR analysis. The ND particles 12, while dispersing in the resin composition 10 or in the thermoplastic resin 11, include surface hydroxy functional groups in such an amount that the proportion of the hydroxy-bonded carbon as a whole is 16.8% or more.

The ND particles 12 in the resin composition 10 have a proportion of carboxy carbon of typically 1.0% or more, preferably 1.2% or more, more preferably 1.5% or more, furthermore preferably 1.7% or more, and still more preferably 2.0% or more, of the totality of carbon contained in the ND particles 12. The upper limit of the proportion of the carboxy carbon is typically 5.0%. As used herein, the term "carboxy carbon" refers to a carbon that is contained in a carboxy group (-C(=O)O including -COOH), where the carboxy group is attached on the basis skeleton of a nanodiamond and present at the surface of the nanodiamond. In the embodiment, the proportion of the carboxy carbon is defined as a value determined by solid-state ¹³C-NMR analysis. The ND particles 12, while dispersing in the resin composition 10 or in the thermoplastic resin 11, include surface carboxy groups in such an amount that the proportion of the carboxy carbon as a whole be typically 1.0% or more.

The ND particles 12 in the resin composition 10 may have a proportion of carbonyl carbon of typically 1.0% or more, preferably 1.2% or more, more preferably 1.5% or more, furthermore preferably 1.7% or more, and still more preferably 2.0% or more, of the totality of carbon contained in the ND particles 12. The upper limit of the proportion of the carbonyl carbon is typically 5.0%. As used herein, the term "carbonyl carbon" refers to a carbon contained in a carbonyl group (-C=O), where the carbonyl group is attached on the basis skeleton of a nanodiamond and present at the surface of the nanodiamond. However, carbon contained in - C(=O)O is not included in the "carbonyl carbon". In the embodiment, the proportion of the carbonyl carbon is defined as a value determined by solid-state ¹³C-NMR analysis. The ND particles 12, while dispersing in the resin composition 10 or in the thermoplastic resin 11, include surface carbonyl functional groups in such an amount that the proportion of the carbonyl carbon as a whole be typically 1.0% or more.

The ND particles 12 in the resin composition 10 may have a total proportion of the hydroxy-bonded carbon and the carboxy carbon of typically 17.8% or more, preferably 18.0% or more, more preferably 18.3%, more preferably 18.5% or more, and furthermore preferably 18.8% or more, of the totality of carbon contained in the ND particles 12. The upper limit of the total proportion is typically 30.0%.

The ND particles 12 in the resin composition 10 may have a total proportion of the hydroxy-bonded carbon and the carbonyl carbon of typically 17.8% or more, preferably 18.0% or more, more preferably 18.3%, furthermore preferably 18.5% or more, and still more preferably 18.8% or more, of the totality of carbon contained in the ND particles 12. The upper limit of the total proportion is typically 30.0%.

The ND particles 12 in the resin composition 10 may have a total proportion of the carboxy carbon and the carbonyl carbon of typically 2.0% or more, preferably 2.2% or more, more preferably 2.4% or more, furthermore preferably 2.7% or more, still more preferably 2.9% or more, and still furthermore preferably 3.2% or more, of the totality of carbon contained in the ND particles 12. The upper limit of the total proportion is typically 8.0%.

The ND particles 12 in the resin composition 10 may have a total proportion of the hydroxy-bonded carbon, the carboxy carbon, and the carbonyl carbon of typically 18.8% or more, preferably 19.0% or more, more preferably 19.2%, furthermore preferably 19.5% or more, still more preferably 19.7% or more, still more preferably 20.0%, and still furthermore preferably 20.5%, of the totality of carbon contained in the ND particles 12. The upper limit of the total proportion is typically 32.0%.

The ND particles 12 in the resin composition 10 may have a proportion of alkenyl carbon of typically 0.1% or more of the totality of carbon contained in the ND particles 12. As used herein, the term "alkenyl carbon" refers to a carbon contained in an alkenyl group, where the alkenyl group is attached on the basic skeleton of a nanodiamond and present on the surface of the nanodiamond.

The ND particles 12 in the resin composition 10 may have a proportion of hydrogen-bonded carbon of typically 20.0% or more of the totality of carbon contained in the ND particles 12. As used herein, the term "hydrogen-bonded carbon" refers to a carbon to which a hydrogen is bonded, where the hydrogen is attached on the basis skeleton of a nanodiamond and present at the surface of the nanodiamond. The bonding of hydrogen as above contributes to stabilized nanodiamond surface carbon.

The ND particles 12 contained in the resin composition 10 may be, for example, detonation nanodiamond particles (nanodiamond particles formed by the detonation technique). Known examples of the detonation technique, which is a nanodiamond production technique, include air-cooling detonation techniques and water-cooling detonation techniques. The ND particles 12 are preferably air-cooled detonation nanodiamond particles. The air-cooled detonation nanodiamond particles tend to include smaller-sized primary particles as compared with water-cooled detonation nanodiamond particles and are advantageous to give a resin composition 10 containing small-sized nanodiamond primary particles in the ND particles 12. The ND particles 12 are more preferably air-cooled atmosphere-coexistent detonation nanodiamond particles, specifically, nanodiamond particles formed by an air-cooling detonation technique performed in the coexistence of a gas having an atmospheric composition (containing oxygen in significant quantity). The air-cooling detonation technique performed in the coexistence of a gas having an atmospheric composition is advantageous for forming nanodiamond particles including large amounts of functional groups on primary particles.

The resin composition 10 may contain one or more other components, in addition to the thermoplastic resin 11 and the ND particles 12. Non-limiting examples of the other components include flame retardants, glass fibers, carbon fibers, antistatic agents, lubricants, and colorants.

The resin composition 10, which has the configuration as described above, can be produced typically in the following manner. Initially, pellets or another material to form the thermoplastic resin 11, the ND particles 12, which are a dry powder, and one or more other components to be added as needed are mixed using a mixing machine (mixing step). Non-limiting examples of the mixing machine include Henschel mixers, tumblers, and planetary centrifugal mills. Next, the mixture resulting from the mixing step is heated and kneaded using a kneading machine. Non-limiting examples of the kneading machine include batch polymer mixers, twinscrew extruders, single-screw extruders, Banbury mixers, and roller mixers. Next, the kneaded material is shaped into a predetermined shape. Alternately, the kneaded material is pelletized. The resulting pellets may be used as a material and subjected to injection molding. Thus, the resin composition 10 can be produced.

The resin composition 10 contains the thermoplastic resin 11 having a melting point of 280°C or higher or a glass transition temperature of 220°C or higher. To soften/melt the thermoplastic resin 11 so as to knead or shape the resin composition 10, heating should be performed up to a high temperature significantly higher than, for example, the glass transition temperature of the thermoplastic resin 11. Even if such heating causes radicals to be generated in the resin composition 10, the decomposition reaction and crosslinking reaction in the thermoplastic resin 11 are restrained by the presence of the ND particles 12 in the resin composition 10, where the reactions will be caused by the action of the radicals. This is probably because as follows. The ND particles 12, which are nanodiamonds, can offer heat resistance even in an environment at a high temperature of higher than 450°C. A graphite layer is assumed to be formed on at least part of, namely on at least the {111} face of, the nanodiamond primary particles each of which is a nanodiamond particle itself or is contained in a nanodiamond particle, where the graphite layer resulting from spontaneous transition from sp³ carbon, which constitutes the diamonds themselves. The presence of sp² carbon in the graphite layer probably contributes in part to trapping/stabilization of radicals, and this probably restrains decomposition and crosslinking, namely, deterioration of the thermoplastic resin 11, where the decomposition and crosslinking will be caused by the radicals. In addition, the restrainment of the crosslinking reaction tends to restrain the thickening of the thermoplastic resin 11, namely, the thickening of the resin composition 10 during working with heating. The restrainment of the decomposition reaction and the crosslinking reaction in the resin composition 10 during working with heating contributes to restraining deterioration of the resin composition 10 in heat resistance and to offering high heat resistance of the resin composition 10, where the resin composition 10 contains the thermoplastic resin 11 having a melting point of 280°C or higher or a glass transition temperature of 220°C or higher. As described above, the resin composition 10 is suitable for offering high heat resistance while restraining deterioration and thickening during working with heating.

In addition, according to the embodiment, the ND particles 12 have, as a surface functional group, hydroxy groups and optionally at least one type of functional group selected from the class consisting of carboxy groups, and carbonyl groups. These surface functional groups are considered to co-operate with sp² carbon present on the nanodiamond surfaces to form a conjugated system, to contribute to trapping and stabilization of radicals, and to thereby contribute to restrainment of the decomposition and crosslinking, namely, deterioration of the thermoplastic resin 11, where the decomposition and crosslinking will be caused by the action of the radicals.

FIG. 2 is a process chart of a method according to an embodiment for producing nanodiamonds which are usable as the ND particles 12 in the resin composition 10. This method includes a forming step S1, a purifying step S2, a chemical deagglutination step S3, a pH-adjustment step S4, a centrifugal separation step S5, and a drying step S6.

In the forming step S1, air-cooling detonation is performed in the coexistence of a gas having an atmospheric composition (containing oxygen in significant quantity), to form nanodiamonds. Initially, a shaped explosive equipped with an electric detonator is placed in a detonation pressure-tight chamber, and the chamber is hermetically sealed so that the explosive is coexistent with a gas having an atmospheric composition at normal atmospheric pressure in the chamber. The chamber is made typically of iron and has a capacity of typically 0.5 to 40 m³, and preferably 2 to 30 m³. The explosive for use herein may be a mixture of trinitrotoluene (TNT) and cyclotrimethylenetrinitroamine, namely, hexogen (RDX). The mass ratio of TNT to RDX is typically from 40:60 to 60:40. The explosive may be used in an amount of typically 0.05 to 2.0 kg, and preferably 0.3 to 1.0 kg.

In the forming step S1, next, the electric detonator is ignited to initiate detonation of the explosive in the chamber. The term "detonation" refers to, among explosions associated with chemical reactions, an explosion in which a flame front travels at a high speed faster than sound, where the reaction occurs on the flame front. In the detonation, the explosive partially undergoes incomplete combustion to liberate carbon, and the liberated carbon serves as a material and forms nanodiamonds by the action of pressure and energy of a shock wave generated by the explosion. In the formation of such nanodiamond products by the detonation technique, initially, adjacent primary particles or crystallites very firmly aggregate by the contribution of not only the van der Waals force, but also Coulomb interaction between crystal faces, to form agglutinates.

In the forming step S1, next, the chamber and the contents thereof are cooled by leaving them stand at room temperature typically for 24 hours. After the natural cooling, a nanodiamond crude product (including nanodiamond agglutinates formed in the above manner, and soot) attached to the chamber inner wall is scraped off using a spatula to collect the nanodiamond crude product. As a result of the air-cooling atmosphere-coexistent detonation technique as described above, the crude product of nanodiamond particles can be obtained. The air-cooling detonation technique performed in the coexistence of a gas having an atmospheric composition (containing oxygen in significant quantity) is advantageous for the formation of nanodiamond particles containing large amounts of surface functional groups on the primary particles. This is provably because as follows. With the air-cooling atmosphere-coexistent detonation technique, growth of diamond nuclei from the material carbon is restrained in a process during which diamond crystallites are formed, and this causes part (some typically with oxygen) of the material carbon to form surface functional groups. The forming step S1 as above, when repeatedly performed in a necessary number of times, can give the nanodiamond crude product in a desired amount.

According to the embodiment, the purifying step S2 includes an acid treatment in which the material nanodiamond crude product is acted upon typically by a strong acid in a water medium. The nanodiamond crude product resulting from the detonation technique tends to include metal oxides. The metal oxides are oxides of metals, such as Fe, Co, and Ni, derived typically from the chamber used in the detonation technique. The metal oxides can be dissolved off from, and removed from, the nanodiamond crude product typically by the action of a predetermined strong acid in a water medium (acid treatment). The strong acid for use in the acid treatment is preferably selected from mineral acids, such as hydrochloric acid, hydrofluoric acid, sulfuric acid, nitric acid, and aqua regia. The acid treatment may employ each of different strong acids alone or in combination. The strong acid(s) may be used in the acid treatment in a concentration of typically 1 to 50 mass percent. The acid treatment may be performed at a temperature of typically 70°C to 150°C for a time of typically 0.1 to 24 hours. The acid treatment can be performed under reduced pressure, or at normal atmospheric pressure, or under pressure (under a load). After the acid treatment as above, the solids (including nanodiamond agglutinates) are washed with water typically by decantation. The water washing of the solids by decantation is preferably repeated until the pH of a sedimentary fluid reaches, for example, 2 to 3.

The purifying step S2, according to the embodiment, also includes an oxidation treatment using an oxidizer so as to remove graphite from the nanodiamond crude product (nanodiamond agglutinates before the completion of purification). The nanodiamond crude product resulting from the detonation technique includes graphite. The graphite is derived from carbon that has not formed nanodiamond crystals, out of carbons liberated from the explosive as a result of partial incomplete combustion. The graphite can be removed from the nanodiamond crude product typically by allowing a predetermined oxidizer to act upon the crude product in a water medium (oxidation treatment), typically after the acid treatment. Non-limiting examples of the oxidizer for use in the oxidation treatment include chromic acid, chromic anhydride, dichromic acid, permanganic acid, perchloric acid, and salts of them. The oxidation treatment may employ each of different oxidizers alone or in combination. The oxidizer(s) may be sued in the oxidation treatment in a concentration of typically 3 to 50 mass percent. The oxidizer may be used in the oxidation treatment in an amount of typically 300 to 500 parts by weight per 100 parts by weight of the nanodiamond crude product to be subjected to the oxidation treatment. The oxidation treatment may be performed at a temperature of typically 100°C to 200°C for a time of typically 1 to 24 hours. The oxidation treatment can be performed under reduced pressure, or at normal atmospheric pressure, or under pressure (under a load). The oxidation treatment is preferably performed in the coexistence of a mineral acid, from the view point of contributing to more efficient graphite removal. Non-limiting examples of the mineral acid include hydrochloric acid, hydrofluoric acid, sulfuric acid, nitric acid, and aqua regia. The mineral acid, when used in the oxidation treatment, may be used in a concentration of typically 5 to 80 mass percent. After the oxidation treatment as above, solids (including nanodiamond agglutinates) are washed with water typically by decantation. The supernatant liquid in the early stages of the water washing is colored. The water washing of the solids by decantation is preferably repeated until a supernatant liquid becomes visually transparent.

Even after purification through the purifying step S2 as above, the detonation nanodiamonds remain in the form of agglutinates (secondary particles), in which primary particles aggregate with very strong interactions therebetween. To deagglutinate (deaggregate) the primary particles from the agglutinates, the chemical deagglutination step S3 is subsequently performed in the method. By allowing the nanodiamond agglutinates to be acted upon typically by a predetermined alkali and hydrogen peroxide in a water medium, the nanodiamond primary particles can be deagglutinated from the nanodiamond agglutinates, and the deagglutination can proceed (deagglutination treatment). Non-limiting examples of the alkali for use in the deagglutination treatment include sodium hydroxide, ammonia, and potassium hydroxide. The alkali is used in the deagglutination treatment in a concentration of preferably 0.1 to 10 mass percent, more preferably 0.2 to 8 mass percent, and furthermore preferably 0.5 to 5 mass percent. The hydrogen peroxide may be used in the deagglutination treatment in a concentration of preferably 1 to 15 mass percent, more preferably 2 to 10 mass percent, and furthermore preferably 4 to 8 mass percent. The deagglutination treatment may be performed at a temperature of typically 40°C to 95°C for a time of typically 0.5 to 5 hours. The deagglutination treatment can be performed under reduced pressure, or at normal atmospheric pressure, or under pressure (under a load). After the deagglutination treatment as above, a supernatant is removed by decantation.

In the method, the pH-adjustment step S4 is subsequently performed. The pH-adjustment step S4 is the step of adjusting the pH of a fluid containing nanodiamonds (nanodiamond-containing fluid), which has undergone the deagglutination treatment, to a predetermined pH before the after-mentioned centrifugal separation treatment. In this step, a sedimentary fluid after decantation is combined with an acid and/or an alkali. The acid for use herein may typically be hydrochloric acid. The adjustment of the pH of the fluid containing nanodiamonds typically to 2 to 3 by this step allows a nanodiamond dispersion prepared by the method to have a pH falling within the range typically of from 4 to 7.

In this method, the centrifugal separation step S5 is subsequently performed. The centrifugal separation step S5 is the step of subjecting the nanodiamond-containing fluid, which has undergone the deagglutination treatment, to a centrifugal separation treatment. Specifically, initially, the nanodiamond-containing fluid undergone the chemical deagglutination step S3 and the pH-adjustment step S4 is subjected to a first centrifugal separation treatment using a centrifugal separator. A supernatant liquid after the first centrifugal separation treatment is often transparent pale yellow. Then, precipitates resulting from the centrifugal separation treatment are separated from the supernatant liquid, suspended in ultrapure water added thereto, and subjected further to another centrifugal separation treatment for solid-liquid separation using a centrifugal separator. The ultrapure water may be added in a volume typically 3 to 5 times the volume of the precipitates. A series of processes including the separation of precipitates from a supernatant liquid after a solid-liquid separation by centrifugal separation, suspending of the precipitates in ultrapure water added thereto, and another centrifugal separation treatment is repeated until a black, transparent supernatant liquid is obtained after a centrifugal separation treatment. When the black, transparent supernatant liquid is obtained after a third or later centrifugal separation treatment, a supernatant liquid obtained after a centrifugal separation treatment between the first centrifugal separation treatment and the third or later centrifugal separation treatment giving the black, transparent supernatant liquid is often colorless, transparent. The black, transparent supernatant liquid obtained as above is a nanodiamond dispersion in which nanodiamond primary particles are present as colloidal particles and are dispersed in water. The centrifugation in the centrifugal separation treatments in this step may be performed at a force of typically 15000 to 25000 × g for a time of typically 10 to 120 minutes. Precipitates formed by, for example, the first centrifugal separation treatment in the centrifugal separation step S5 as above contains nanodiamond secondary particles in a larger amount as compared with primary particles. The precipitates, after separated from the supernatant liquid, are suspended in ultrapure water added thereto, and yield a nanodiamond-containing fluid. Separately, precipitates formed through any of centrifugal separation treatments, such as precipitates remained after separation of the black, transparent supernatant liquid therefrom, may be subjected again to a series of processes including the chemical deagglutination step S3, the pH-adjustment step S4, and the centrifugal separation step S5, alone or in combination with other solids (including nanodiamond agglutinates), which have undergone the purifying step S2.

The nanodiamonds in the product resulting from the detonation technique are initially in the form of agglutinates (secondary particles) in which primary particles very firmly interact and aggregate with each other, as described above. In the process from the forming step S1 to the centrifugal separation step S5, the nanodiamond agglutinates are acted upon not only by a chemical species under heating conditions in the purifying step S2, but also by a chemical species under heating conditions in the chemical deagglutination step S3. This chemically loosens, in the chemical deagglutination step S3, the agglutination between at least part of nanodiamond primary particles in the nanodiamond agglutinates and allows the nanodiamond primary particles to be more easily separated from the nanodiamond agglutinates. In the centrifugal separation step S5, the nanodiamond-containing fluid as above is repeatedly subjected to solid-liquid separation using the action of centrifugal force, and to a subsequent suspending operation. After centrifugal separation treatments in a predetermined number of times, a black, transparent supernatant liquid containing nanodiamond primary particles as dispersed is collected. Thus, a nanodiamond dispersion, in which nanodiamond primary particles are dispersed as colloidal particles, can be prepared. The prepared nanodiamond dispersion can have a higher nanodiamond concentration by reducing the water content. The water content can be reduced typically using an evaporator.

In the method, the drying step S6 is subsequently performed. Specifically, the nanodiamond dispersion or nanodiamond-containing fluid resulting from the centrifugal separation step S5 as above is subjected to a drying treatment to give a nanodiamond dry powder. Non-limiting examples of the drying treatment technique include spray drying using a spray dryer, and evaporation to dryness using an evaporator.

Thus, nanodiamonds which are usable as the ND particles 12 in the resin composition 10 can be produced.

In the method, the deagglutination treatment and the centrifugal separation treatment including the suspending operation as above are performed so as to separate the nanodiamond primary particles from the nanodiamond agglutinates (secondary particles). The chemical deagglutination step S3 herein does not employ conventional bead milling using zirconia beads as a deagglutination technique to separate the nanodiamond primary particles. The nanodiamond dispersion and the nanodiamond powder prepared by the method are approximately devoid of zirconia. Assume that the nanodiamond dispersion and the nanodiamond powder are subjected to detection of zirconia. Even in this case, it is supposed that zirconia in an amount larger than the detection limit of an employed detection method is not detected. The absence of zirconia as above is advantageous for giving nanodiamonds with higher purity.

The embodiment may employ a physical deagglutination technique instead of the above-described deagglutination technique in the chemical deagglutination step S3, so as to deagglutinate the nanodiamond agglutinates, which have undergone the purifying step S2, into nanodiamond primary particles. Non-limiting examples of the physical deagglutination technique include techniques using any of high-shear mixers, homomixers, ball mills, bead mills, highpressure homogenizers, ultrasonic homogenizers, and colloid mills. The method, when employing the physical deagglutination technique, preferably employs bead milling using a bead mill. When the deagglutination treatment is performed by such a physical deagglutination technique, coarse particles are removed from a nanodiamond-containing slurry resulting from the deagglutination treatment. For example, coarse particles, such as nanodiamond coarse particles, can be removed from the slurry typically by a classification operation using centrifugal separation with a classifier. This gives a black, transparent nanodiamond dispersion in which nanodiamond primary particles are dispersed as colloidal particles.

### Examples

The present invention will be illustrated in further detail with reference to several examples below. It should be noted, however, that the examples are by no means intended to limit the scope of the present invention.

### Nanodiamond Production Example 1

A nanodiamond dry powder was produced through a forming step, a purifying step, a chemical deagglutination step, a pH-adjustment step, a centrifugal separation step, and a drying step as described below.

In the forming step, initially, a shaped explosive equipped with an electric detonator was placed in a detonation pressure-tight chamber, and the chamber was hermetically sealed so that the explosive was coexistent with a gas having an atmospheric composition at normal atmospheric pressure in the chamber. The chamber was made of iron and had a capacity of 15 m³. The explosive used herein was 0.50 kg of a mixture of trinitrotoluene (TNT) and cyclotrimethylenetrinitroamine, namely, hexogen (RDX). The mass ratio of TNT to RDX in the explosive was 50:50. Next, the electric detonator was ignited to initiate detonation of the explosive in the chamber. The chamber and the contents thereof were then cooled by leaving stand at room temperature for 24 hours. After the natural cooling, a nanodiamond crude product (including nanodiamond agglutinates formed by the detonation technique, and soot) attached to the chamber inner wall was scraped off using a spatula to collect the nanodiamond crude product. The nanodiamond crude product was collected in an amount of 0.025 kg.

Next, the nanodiamond crude product (nanodiamond crude product P') resulting from the above forming step performed in multiple times was subjected to an acid treatment in the purifying step. Specifically, 200 g of the nanodiamond crude product were combined with 6 L of 10 mass percent hydrochloric acid to give a slurry, and the slurry was subjected to a heat treatment under reflux at normal atmospheric pressure for one hour. The heating in the acid treatment was performed at a temperature of 85°C to 100°C. Next, after being cooled, solids (including nanodiamond agglutinates and soot) were washed with water by decantation. The water washing of the solids by decantation was repeated until the pH of a sedimentary fluid became from a lower level up to 2.

Next, an oxidation treatment in the purifying step was performed. Specifically, the sedimentary fluid after decantation was combined with 5 L of a 60 mass percent aqueous sulfuric acid solution and 2 L of a 60 mass percent aqueous chromic acid solution to give a slurry, and the slurry was subjected to a heat treatment under reflux at normal atmospheric pressure for 5 hours. The heating in the oxidation treatment was performed at a temperature of 120°C to 140°C. Next, after being cooled, solids (including nanodiamond agglutinates) were washed with water by decantation. A supernatant liquid in the early stages of the water washing was colored. The water washing of the solids by decantation was repeated until a supernatant liquid became visually transparent.

Next, the chemical deagglutination step was performed. Specifically, the sedimentary fluid after decantation was combined with 1 L of a 10 mass percent aqueous sodium hydroxide solution and 1 L of a 30 mass percent aqueous hydrogen peroxide solution to give a slurry, and the slurry was subjected to a heat treatment under reflux at normal atmospheric pressure for one hour (chemical deagglutination treatment). The heating in the chemical deagglutination treatment was performed at a temperature of 50°C to 105°C. Next, after cooling, a supernatant was removed by decantation.

Next, the pH-adjustment step was performed. Specifically, a sedimentary fluid resulting from decantation after the chemical deagglutination treatment was combined with hydrochloric acid to adjust the pH of the sedimentary fluid to 2.5. This gave a slurry whose pH was adjusted.

Next, the centrifugal separation step was performed. Specifically, the slurry (nanodiamond-containing fluid) undergone the pH adjustment was initially subjected to a first centrifugal separation treatment using a centrifugal separator. The centrifugation in the centrifugal separation treatment was performed at a force of 20000 × g for a time of 10 minutes. A supernatant liquid after the first centrifugal separation treatment was transparent pale yellow. In this step, next, precipitates resulting from the first centrifugal separation treatment were separated from the supernatant liquid, suspended in ultrapure water added thereto, and subjected to a second centrifugal separation treatment for solid-liquid separation using a centrifugal separator. The ultrapure water was added in a volume 4 times the volume of the precipitates. The centrifugation in the second centrifugal separation treatment was performed at a force of 20000 × g for a time of 60 minutes. A supernatant liquid after the second centrifugal separation treatment was colorless, transparent. In this step, precipitates resulting from the second centrifugal separation treatment were then separated from the supernatant liquid, suspended in ultrapure water added thereto, and subjected to a third centrifugal separation treatment for solid-liquid separation using a centrifugal separator. The ultrapure water was added in a volume 4 times the volume of the precipitates. The centrifugation in the third centrifugal separation treatment was performed at a force of 20000 × g for a time of 60 minutes. A supernatant liquid after the third centrifugal separation treatment was black, transparent. A series of processes was then repeated as long as a black, transparent supernatant liquid was obtained after a centrifugal separation treatment, where the processes include separation of precipitates from a supernatant liquid after a solid-liquid separation by centrifugal separation; suspending of the precipitates by addition of a quadruple amount of ultrapure water; and another (further) centrifugal separation treatment (at a centrifugal force of 20000 × g for a centrifugal time of 60 minutes).

A black, transparent nanodiamond dispersion was obtained in the above manner. The pH of the resulting nanodiamond dispersion after the third centrifugal separation treatment was determined using a pH test paper (trade name Three Band pH Test Paper, supplied by AS ONE Corporation) and found to be 6. This dispersion had a nanodiamond solids concentration of 1.08 mass percent. The dispersion was subjected to measurement of nanodiamond particles contained in the dispersion by dynamic light scattering technique and found to have a particle diameter D50 (median diameter) of 6.04 nm. Part of the dispersion was diluted with ultrapure water to a nanodiamond concentration of 0.2 mass percent to give a nanodiamond dispersion specimen. The zeta potential of nanodiamond particles in the specimen was then measured and found to be -42 mV (25°C, pH 6).

Next, the drying step was performed. Specifically, the nanodiamond dispersion obtained in the above manner was subjected to spray drying into a powder, using a spray dryer (trade name Spray Dryer B-290, supplied by Nihon BUCHI K.K.). Thus, a nanodiamond dry powder (nanodiamond powder P₁) was produced.

The nanodiamond powder P₁ was subjected to crystallography using an X-ray diffractometer (trade name SmartLab, supplied by Rigaku Corporation). As a result, an intense diffraction peak was observed at a diamond diffraction peak position, namely, a diffraction peak position from the (111) face of a diamond crystal, on the basis of which the powder obtained in the above manner was identified to be diamond. The nanodiamond powder P₁ was also subjected to small-angle X-ray scattering measurement using an X-ray diffractometer (trade name SmartLab, supplied by Rigaku Corporation). On the basis of this, the nanodiamond primary particle diameter was estimated in a region at scattering angles of 1° to 3° using a particle diameter distribution analysis software (trade name NANO-Solver, supplied by Rigaku Corporation). The estimation was performed assuming that nanodiamond primary particles are spherical and are present at a particle density of 3.51 g/cm³. As a result of the measurement, the average particle diameter of nanodiamond primary particles was found to be 4.240 nm, and the relative standard deviation (RSD) on primary particle distribution was found to be 41.4%. The data demonstrated that a group of relatively small primary particles was present in a relatively sharp distribution, where the primary particles had a smaller average particle diameter as compared with the particle diameter D50 (6.04 nm) determined by dynamic light scattering technique.

The nanodiamond powder P₁ was subjected to solid-state ¹³C-NMR analysis in a manner as described below. The resulting ¹³C DD/MAS NMR spectrum of the analyte gave, as observed peaks, a peak derived from a sp³ carbon as a nanodiamond principal component; a peak derived from a carbon to which a hydroxy group is bonded (-COH); a peak derived from a carbon contained in a carboxy group (-C(=O)O including -COOH); a peak derived from a carbon contained in a carbonyl group (-C=O); a peak derived from a carbon contained in an alkenyl group (C=C); and a peak derived from a carbon to which a hydrogen is bonded (-CH). The proportions of these carbons calculated after performing waveform separation on each peak were 54.5% for the sp³ carbon, 17.0% for the hydroxy-bonded carbon, 1.7% for the carboxy carbon, 1.5% for the carbonyl carbon, 0.2% for the alkenyl carbon, and 25.1% for the hydrogen-bonded carbon. Apart from this, a nanodiamond dry powder was prepared from a commercially available aqueous nanodiamond dispersion (trade name Vox D, supplied by Carbodeon) including nanodiamond particles being dispersed. This dispersion has a nanodiamond concentration of 5 mass percent, a particle diameter D50 of 5 nm, and a zeta potential of -55 mV at pH 9. This nanodiamond dry powder was subjected to solid-state ¹³C-NMR analysis. The resulting ¹³C DD/MAS NMR spectrum of the analyte gave, as observed peaks, a peak derived from sp³ carbon as a nanodiamond principal component; a peak derived from a carbon to which a hydroxy group is bonded (-COH); a peak derived from a carbon contained in a carboxy group (-C(=O)O including -COOH); a peak derived from a carbon contained in a carbonyl group (-C=O); and a peak derived from a carbon to which a hydrogen atom is bonded (-CH). However, no peak derived from a carbon contained in an alkenyl group (C=C) was observed. The proportions of these carbons calculated after performing waveform separation on each peak were 66.5% for the sp³ carbon, 16.1% for the hydroxy-bonded carbon, 0.2% for the carboxy carbon, 0.4% for the carbonyl carbon, and 16.8% for the hydrogen-bonded carbon.

The nanodiamond powder P₁ was subjected to inductively coupled plasma (ICP) emission spectrometry as described below, in order to measure the content of zirconia. However, zirconia was not detected. Specifically, no measurement result of the zirconia content higher than the detection limit (lower limit) of 50 ppm by mass was obtained.

### Nanodiamond Production Example 2

An acid treatment and an oxidation treatment in a purifying step, a chemical deagglutination step, and a pH-adjustment step were performed by procedures similar to those in Production Example 1, except for using an air-cooled detonation nanodiamond soot obtained by air-cooling detonation (supplied by ALIT) instead of the nanodiamond crude product P'. Then a centrifugal separation step was performed. Specifically, a slurry (nanodiamond-containing fluid) undergone the pH-adjustment step was subjected to a centrifugal separation treatment using a centrifugal separator. The centrifugation in the centrifugal separation treatment was performed at a force of 20000 × g for a time of 10 minutes. Next, precipitates resulting from the centrifugal separation treatment were separated from a supernatant liquid, and suspended in ultrapure water added thereto, where the ultrapure water was added in a volume 4 times the volume of the precipitates. This gave a fluid containing nanodiamond particles undergone the processes such as purification. Next, a drying step was performed. Specifically, the nanodiamond-containing fluid was subjected to spray drying into a powder, using a spray dryer (trade name Spray Dryer B-290, supplied by Nihon BUCHI K.K.). Thus, a nanodiamond dry powder (nanodiamond powder P₂) was produced.

The nanodiamond powder P₂ was subjected to crystallography using an X-ray diffractometer (trade name SmartLab, supplied by Rigaku Corporation) as with the nanodiamond powder P₁, and identified to be diamond. In addition, as with the nanodiamond powder P₁, the nanodiamond powder P₂ was subjected to small-angle X-ray scattering measurement using an X-ray diffractometer (trade name SmartLab, supplied by Rigaku Corporation), on the basis of which the nanodiamond primary particle diameter was estimated in a region of scattering angles of 1° to 3° using a particle diameter distribution analysis software (trade name NANO-Solver, supplied by Rigaku Corporation). As a result of the measurement, the average particle diameter of nanodiamond primary particles was found to be 5.945 nm, and the relative standard deviation (RSD) on primary particle distribution was found to be 38.1%.

The nanodiamond powder P₂ was subjected to solid-state ¹³C-NMR analysis in a manner as described below. The resulting ¹³C DD/MAS NMR spectrum of the analyte gave, as observed peaks, a peak derived from a sp³ carbon as a nanodiamond principal component; a peak derived from a carbon to which a hydroxy group is bonded (-COH); a peak derived from a carbon contained in a carboxy group (-C(=O)O including -COOH); a peak derived from a carbon contained in a carbonyl group (-C=O); and a peak derived from a carbon to which a hydrogen atom is bonded (-CH). The proportions of these carbons calculated after performing waveform separation on each peak were 70.0% for the sp³ carbon, 14.7% for the hydroxy-bonded carbon, 0.5% for the carboxy carbon, 0.4% for the carbonyl carbon, and 14.4% for the hydrogen-bonded carbon.

The nanodiamond powder P₂ was subjected to ICP emission spectrometry as described below, in order to measure the content of zirconia. However, zirconia was not detected. Specifically, no measurement result of the zirconia content higher than the detection limit (lower limit) of 50 ppm by mass was obtained.

### Solids Concentration

The solids concentration of an analyte nanodiamond dispersion was calculated from: a weight determined by weighing 3 to 5 g of the dispersion; and a weight determined by heating and thereby evaporating the weighed dispersion to remove water therefrom and to leave dry matter (powder), and weighing the dry matter using a precision balance.

### Median Diameter

The particle diameter D50 (median diameter) of nanodiamond particles contained in an analyte nanodiamond dispersion was a value measured by dynamic light scattering technique (noncontact backscatter mode) using an apparatus Zetasizer Nano ZS (trade name) supplied by Spectris Co., Ltd. Before the measurement, a sample nanodiamond dispersion was diluted with ultrapure water to a nanodiamond concentration of 0.5 to 2.0 mass percent, and then exposed to ultrasound using an ultrasonic cleaner, to give the analyte.

### Zeta Potential

The zeta potential of nanodiamond particles contained in an analyte nanodiamond dispersion was a value measured by laser Doppler electrophoresis using an apparatus Zetasizer Nano ZS (trade name) supplied by Spectris Co., Ltd. Before the measurement, a sample nanodiamond dispersion was diluted with ultrapure water to a nanodiamond concentration of 0.2 mass percent, and exposed to ultrasound using a ultrasonic cleaner, to give the analyte. The pH of the nanodiamond dispersion subjected to the measurement was a value measured using a pH test paper (trade name Three Band pH Test Paper, supplied by AS ONE Corporation).

### Solid-State ¹³C-NMR Analysis

The solid-state ¹³C-NMR analysis was performed by solid-state NMR using a solid-state NMR system (trade name CMX-300 Infinity, supplied by CHEMAGNETICS, Inc.). The technique and other factors, and conditions for the measurement are as follows.
Measurement method: DD/MAS
Measurement nuclear frequency: 75.188829 MHz (¹³C nuclei)
Spectrum width: 30.003 kHz
Pulse width: 4.2 µsec (90° pulse)
Pulse repetition time: ACQTM 68.26 msec, PD 15 sec
Measurement points: 2048 (data points: 8192)
Reference material: polydimethylsiloxane (external reference: 1.56 ppm)
Temperature: room temperature (about 22°C)
Sample spinning frequency: 8.0 kHz

### ICP Emission Spectrometry

A sample nanodiamond dispersion or nanodiamond-containing fluid was heated and thereby evaporated to remove water therefrom and to leave dry matter (powder), and 100 mg of the dry matter were placed in a porcelain crucible and, as intact, subjected to dry decomposition in an electric furnace. The dry decomposition was performed in three stages, namely, at 450°C for one hour, subsequently at 550°C for one hour, and subsequently at 650°C for one hour. The residue in the porcelain crucible after the dry decomposition as above was combined with 0.5 ml of concentrated sulfuric acid and evaporated to dryness. The resulting residue remaining after evaporation to dryness was finally dissolved in 20 ml of ultrapure water. Thus, an analyte was prepared. The analyte was subjected to ICP emission spectrometry using an ICP emission spectrophotometer (trade name CIROS120, supplied by Rigaku Corporation). The analyte was prepared so that the detection lower limit in this analysis be 50 ppm by mass. Standard solutions for calibration curve plotting used in the analysis were prepared by appropriately diluting a mixed standard solution XSTC-22 (supplied by SPEX CertiPrep) and an atomic absorption analysis standard solution Zr1000 (supplied by Kanto Chemical Co., Inc.) each with an aqueous sulfuric acid solution having a concentration identical to the sulfuric acid concentration of the analyte. In the analysis, the concentration of zirconia in the analyte was determined by performing an operation and analysis as above on a blank crucible to give a measured value, and subtracting this measured value (blank) from the measured value on the nanodiamond dispersion analyte, which is a measurement object.

### Example 1

A mixture (30 g in total) of 100 parts by weight of a poly(ether ether ketone) (PEEK) (trade name VESTAKEEP L4000G, supplied by Daicel-Evonik Ltd.), which is a highly heat-resistant thermoplastic resin, and 0.5 part by weight of the nanodiamond powder P₁ was kneaded using a kneading/extrusion molding evaluation testing machine (trade name LABO PLASTOMILL R-30, having a capacity of kneading chamber of 30 cc, supplied by Toyo Seiki Seisaku-Sho Ltd.), and yielded a resin composition according to Example 1. The kneading was performed at a temperature of 400°C and a number of revolutions of rollers in the kneading chamber of 60 rpm for a time of 15 minutes. The resin composition according to Example 1 was subjected to dynamic viscoelastic measurement using a rotary rheometer (trade name MCR 302, supplied by Anton Paar GmbH), and the value of complex viscosity η* (Pa-s) determined by the measurement was monitored. The measurement was performed using parallel plates having a diameter of 8 mm at a measurement temperature of 400°C, a frequency of 1 Hz, and a strain to be applied to the sample of 3%. On Example 1, a complex viscosity measured one minute later and a complex viscosity measured one hour later, each from the start of the measurement, and a thickening ratio are given in Table 1, where the thickening ratio is a value determined by dividing the complex viscosity measured one hour later by the complex viscosity measured one minute later.

### Example 2 (Reference)

A resin composition according to Example 2 was prepared by kneading a mixture by a procedure similar to that in Example 1, except for using, to form the mixture, 0.5 part by weight of the nanodiamond powder P₂ instead of the nanodiamond powder P₁ (0.5 part by weight). The resin composition according to Example 2 was subjected to dynamic viscoelastic measurement to monitor a complex viscosity η* (Pa·s), as with the resin composition according to Example 1. On Example 2, a complex viscosity measured one minute later and a complex viscosity measured one hour later each from the start of the measurement, and a thickening ratio are given in Table 1, where the thickening ratio is a value determined by dividing the complex viscosity measured one hour later by the complex viscosity measured one minute later.

### Comparative Example 1

A resin composition according to Comparative Example 1 was prepared by kneading 30 g of the same PEEK (trade name VESTAKEEP L4000G) as used in Example 1, using the kneading/extrusion molding evaluation testing machine (trade name LABO PLASTOMILL R-30, supplied by Toyo Seiki Seisaku-Sho Ltd.). The resin composition according to Comparative Example 1 was subjected to dynamic viscoelastic measurement to monitor a complex viscosity η* (Pa·s), as with the resin composition according to Example 1. On Comparative Example 1, a complex viscosity measured one minute later and a complex viscosity measured one hour later each from the start of the measurement, and a thickening ratio are given in Table 1, where the thickening ratio is a value determined by dividing the complex viscosity measured one hour later by the complex viscosity measured one minute later.

### Comparative Example 2

A resin composition according to Comparative Example 2 was prepared by kneading a mixture by a procedure similar to that in Example 1, except for using 0.5 part by weight of a hindered phenolic compound (trade name IRGANOX 1010FF, supplied by BASF SE) instead of the nanodiamond powder P₁ (0.5 part by weight). The resin composition according to Comparative Example 2 was subjected to dynamic viscoelastic measurement to monitor a complex viscosity η* (Pa·s), as with the resin composition according to Example 1. On Comparative Example 2, a complex viscosity measured one minute later and a complex viscosity measured one hour later each from the start of the measurement, and a thickening ratio are given in Table 1, where the thickening ratio is a value determined by dividing the complex viscosity measured one hour later by the complex viscosity measured one minute later.

### Example 3 (Reference)

A resin composition according to Example 3 was prepared by kneading a mixture (30 g in total) of 100 parts by weight of a poly(phenylene sulfide) (PPS) (trade name DURAFIDE 0220A9, supplied by Polyplastics Co., Ltd.), which is a highly heat-resistant thermoplastic resin, and 0.5 part by weight of the nanodiamond powder P₂, using a kneading/extrusion molding evaluation testing machine (trade name LABO PLASTOMILL R-30, having a kneading chamber capacity of 30 cc, supplied by Toyo Seiki Seisaku-Sho Ltd.). The kneading was performed at a temperature of 330°C and a number of revolutions of rollers in the kneading chamber of 60 rpm for a time of 15 minutes. The resin composition according to Example 3 was subjected to dynamic viscoelastic measurement using a rotary rheometer (trade name MCR 302, supplied by Anton Paar GmbH) to monitor a complex viscosity η* (Pa·s) determined by the measurement. The measurement was performed using parallel plates having a diameter of 8 mm at a measurement temperature of 330°C, a frequency of 1 Hz, and a strain to be applied to the sample of 3%. On Example 3, a complex viscosity measured one minute later and a complex viscosity measured one hour later each from the start of the measurement, and a thickening ratio are given in Table 2, where the thickening ratio is a value determined by dividing the complex viscosity measured one hour later by the complex viscosity measured one minute later.

### Comparative Example 3

A resin composition according to Comparative Example 3 was prepared by kneading 30 g of the same PPS (trade name DURAFIDE 0220A9) as used in Example 3, using the kneading/extrusion molding evaluation testing machine (trade name LABO PLASTOMILL R-30, supplied by Toyo Seiki Seisaku-Sho Ltd.). The resin composition according to Comparative Example 3 was subjected to dynamic viscoelastic measurement to monitor a complex viscosity η* (Pa·s), as with the resin composition according to Example 3. On Comparative Example 3, a complex viscosity measured one minute later and a complex viscosity measured one hour later each from the start of the measurement, and a thickening ratio are given in Table 2, where the thickening ratio is a value determined by dividing the complex viscosity measured one hour later by the complex viscosity measured one minute later.

### Comparative Example 4

A resin composition according to Comparative Example 4 was prepared by kneading 30 g of a mixture by a procedure similar to that in Example 3, except for using, to form the mixture, 0.5 part by weight of a hindered phenolic compound (trade name IRGANOX 1010FF, supplied by BASF SE) instead of the nanodiamond powder P₂ (0.5 part by weight). The resin composition according to Comparative Example 4 was subjected to dynamic viscoelastic measurement to monitor a complex viscosity η* (Pa·s), as with the resin composition according to Example 3. On Comparative Example 4, a complex viscosity measured one minute later and a complex viscosity measured one hour later each from the start of the measurement, and a thickening ratio are given in Table 2, where the thickening ratio is a value determined by dividing the complex viscosity measured one hour later by the complex viscosity measured one minute later.

### Evaluations

The results presented in Table 1 demonstrate that the resin composition according to Example 1 (thickening ratio: 1.25), which is a kneaded material of the PEEK with the nanodiamond powder P₁, and the resin composition according to Example 2 (thickening ratio: 1.29), which is a kneaded material of the PEEK with the nanodiamond powder P₂, less undergo thickening in a high-temperature environment, as compared with the resin composition according to Comparative Example 1 (thickening ratio: 1.83), which is a kneaded material of the PEEK alone, and the resin composition according to Comparative Example 2 (thickening ratio: 1.81), which is a kneaded material of the PEEK and the hindered phenolic compound. On the basis of a comparison between Comparative Example 1 and Comparative Example 2, it is evaluated that the hindered phenolic compound in the resin composition according to Comparative Example 2 approximately fails to effectively restrain thickening. This is probably because the hindered phenolic compound fails to endure high-temperature environments during the course of the kneading at 400°C to give the resin composition according to Comparative Example 2, and during the course of the dynamic viscoelastic measurement at 400°C performed on the resin composition according to Comparative Example 2. The thickening-restraining effect in the resin composition according to Example 1 indicates high heat resistance of the nanodiamond powder P₁ or nanodiamond particles in a high-temperature environment at 400°C. The thickening-restraining effect in the resin composition according to Example 2 indicates high heat resistance of the nanodiamond powder P₂ or nanodiamond particles in a high-temperature environment at 400°C.

The results presented in Table 2 demonstrate that the resin composition according to Example 3 (thickening ratio: 1.27), which is a kneaded material of the PPS with the nanodiamond powder P₂ in combination, less undergoes thickening in a high-temperature environment, as compared with the resin composition according to Comparative Example 3 (thickening ratio: 1.43), which is a kneaded material of the PPS alone, and the resin composition according to Comparative Example 4 (thickening ratio: 1.42), which is a kneaded material of the PPS with the hindered phenolic compound. On the basis of a comparison between Comparative Example 3 and Comparative Example 4, it is evaluated that the hindered phenolic compound in the resin composition according to Comparative Example 4 approximately fails to effectively restrain thickening. This is probably because the hindered phenolic compound fails to endure high-temperature environments during the course of the kneading at 330°C to give the resin composition according to Comparative Example 4, and during the course of the dynamic viscoelastic measurement at 330°C performed on the resin composition according to Comparative Example 4. The thickening-restraining effect in the resin composition according to Example 3 indicates high heat resistance of the nanodiamond powder P₂ or nanodiamond particles in a high-temperature environment at 330°C.

**[Table 1]**

| | Composition | | Complex viscosity one minute later (Pa·s) | Complex viscosity one hour later (Pa·s) | Thickening ratio |
|---|---|---|---|---|---|
| Example 1 | PEEK | 100 parts by weight | 15800 | 19800 | 1.25 |
| | Nanodiamond (P₁) | 0.5 part by weight | | | |
| Example 2 | PEEK | 100 parts by weight | 15600 | 20100 | 1.29 |
| (Reference) | Nanodiamond (P₂) | 0.5 part by weight | | | |
| Comparative Example 1 | PEEK | | 15200 | 27800 | 1.83 |
| Comparative Example 2 | PEEK | 100 parts by weight | 16900 | 30600 | 1.81 |
| | IRGANOX 1010FF | 0.5 part by weight | | | |

**[Table 2]**

| | Composition | | Complex viscosity one minute later (Pa·s) | Complex viscosity one hour later (Pa-s) | Thickening ratio |
|---|---|---|---|---|---|
| Example 3 (Reference) | PPS | 100 parts by weight | 260 | 330 | 1.27 |
| | Nanodiamond (P₂) | 0.5 part by weight | | | |
| Comparative Example 3 | PPS | | 259 | 370 | 1.43 |
| Comparative Example 4 | PPS | 100 parts by weight | 263 | 373 | 1.42 |
| | IRGANOX 1010FF | 0.5 part by weight | | | |

### Reference Signs List

- 10: resin composition
- 11: thermoplastic resin
- 12: ND particle (nanodiamond particle)
- S1: forming step
- S2: purifying step
- S3: chemical deagglutination step
- S4: pH-adjustment step
- S5: centrifugal separation step
- S6: drying step

## Claims

1. A resin composition comprising:
a thermoplastic resin having a melting point of 280°C or higher or a glass transition temperature of 220°C or higher; and
nanodiamond particles,
wherein the nanodiamond particles comprise surface hydroxy groups, and wherein the nanodiamond particles have a proportion of hydroxy-bonded carbon of 16.8% or more of the totality of carbon in the nanodiamond particles.

2. The resin composition according to claim 1, wherein the nanodiamond particles are present in an amount of 0.001 to 5 parts by weight per 100 parts by weight of the thermoplastic resin.

3. The resin composition according to one of claims 1 and 2, wherein the nanodiamond particles are detonation nanodiamond particles.

4. The resin composition according to any one of claims 1 to 3, wherein the nanodiamond particles comprise surface carboxy groups.

5. The resin composition according to any one of claims 1 to 4, wherein the nanodiamond particles comprise surface carbonyl groups.

6. The resin composition according to any one of claims 1 to 5, wherein the nanodiamond particles have a median diameter of 10 µm or less.

7. The resin composition according to any one of claims 1 to 6, wherein the thermoplastic resin comprises at least one of an aromatic poly(ether ketone) and a poly(phenylene sulfide).

8. The resin composition according to claim 7, wherein the aromatic poly(ether ketone) comprises at least one selected from the class consisting of:
poly(ether ketone)s;
poly(ether ether ketone)s;
poly(ether ketone ketone)s; and
poly(ether ether ketone ketone)s.

## Patentansprüche

1. Harzzusammensetzung, umfassend:
ein thermoplastisches Harz mit einem Schmelzpunkt von 280°C oder höher oder einer Glasübergangstemperatur von 220°C oder höher; und
Nanodiamantteilchen,
wobei die Nanodiamantteilchen Oberflächenhydroxygruppen umfassen und wobei die Nanodiamantteilchen einen Anteil an hydroxygebundenem Kohlenstoff von 16,8% oder mehr an der Gesamtheit des Kohlenstoffs in den Nanodiamantteilchen aufweisen.

2. Harzzusammensetzung nach Anspruch 1, wobei die Nanodiamantteilchen in einer Menge von 0,001 bis 5 Gewichtsteilen pro 100 Gewichtsteile des thermoplastischen Harzes vorhanden sind.

3. Harzzusammensetzung nach einem der Ansprüche 1 und 2, wobei die Nanodiamantteilchen Detonations-Nanodiamantteilchen sind.

4. Harzzusammensetzung nach einem der Ansprüche 1 bis 3, wobei die Nanodiamantteilchen Oberflächencarboxygruppen umfassen.

5. Harzzusammensetzung nach einem der Ansprüche 1 bis 4, wobei die Nanodiamantteilchen Oberflächencarbonylgruppen umfassen.

6. Harzzusammensetzung nach einem der Ansprüche 1 bis 5, wobei die Nanodiamantteilchen einen medianen Durchmesser von 10 µm oder weniger aufweisen.

7. Harzzusammensetzung nach einem der Ansprüche 1 bis 6, wobei das thermoplastische Harz mindestens eines aus einem aromatischen Poly(etherketon) und einem Poly(phenylensulfid) umfasst.

8. Harzzusammensetzung nach Anspruch 7, wobei das aromatische Poly(etherketon) mindestens eines umfasst, das ausgewählt ist aus der Klasse bestehend aus:
Poly(etherketon)en
Poly(etheretherketon)en
Poly(etherketonketon)en; und
Poly(etheretherketonketon)en.

## Revendications

1. Composition de résine comprenant :
une résine thermoplastique ayant un point de fusion de 280 °C ou supérieur ou une température de transition vitreuse de 220 °C ou supérieure ; et
des particules de nanodiamant,
dans laquelle les particules de nanodiamant comprennent des groupes hydroxyle de surface, et dans laquelle les particules de nanodiamant ont une proportion de carbone lié à de l'hydroxyle de 16,8 % ou plus de la totalité du carbone dans les particules de nanodiamant.

2. Composition de résine selon la revendication 1, dans laquelle les particules de nanodiamant sont présentes à hauteur de 0,001 à 5 parties en poids pour 100 parties en poids de la résine thermoplastique.

3. Composition de résine selon l'une des revendications 1 et 2, dans laquelle les particules de nanodiamant sont des particules de nanodiamant de détonation.

4. Composition de résine selon l'une quelconque des revendications 1 à 3, dans laquelle les particules de nanodiamant comprennent des groupes carboxyle de surface.

5. Composition de résine selon l'une quelconque des revendications 1 à 4, dans laquelle les particules de nanodiamant comprennent des groupes carbonyle de surface.

6. Composition de résine selon l'une quelconque des revendications 1 à 5, dans laquelle les particules de nanodiamant ont un diamètre médian de 10 µm ou moins.

7. Composition de résine selon l'une quelconque des revendications 1 à 6, dans laquelle la résine thermoplastique comprend au moins l'un(e) parmi une poly(éther-cétone) aromatique et un poly(sulfure de phénylène).

8. Composition de résine selon la revendication 7, dans laquelle la poly(éther cétone) aromatique comprend au moins un composé choisi dans la catégorie constituée par des :
poly(éther-cétone) ;
poly(éther-éther-cétone) ;
poly(éther-cétone-cétone) ; et
poly(éther-éther-cétone-cétone).
